# EUROPEAN PATENT SPECIFICATION

(11) **EP 1 915 186 B1**
(45) Date of publication and mention of the grant of the patent: **21.12.2011**
(21) Application number: 06745115.3
(22) Date of filing: 24.05.2006
(51) Int. Cl.: A61M 11/00, A61H 23/02, A61H 15/00

(54) **A VIBRATING DEVICE FOR TREATING NASAL CONGESTION AND SINUSITIS SYMPTOMS**
VIBRIERVORRICHTUNG ZUR BEHANDLUNG VON NASALER KONGESTION UND SINUSITIS-SYMPTOMEN
DISPOSITIF VIBRANT PERMETTANT DE TRAITER LA CONGESTION NASALE ET LES SYMPTOMES DE LA SINUSITE

(30) Priority: 02.06.2005 IL 16897405
(43) Date of publication of application: 30.04.2008
(73) Proprietor: ADS&B Investment Fund L.P., Herzliya Pituach 46733 (IL)
(72) Inventor: AVNI, Yuval, 53259 Giv'ataim (IL)
(74) Representative: Lecomte, Didier
(86) International application number: PCT/IL2006/000612
(87) International publication number: WO 2006/129305

(56) References cited:
- WO-A1-93/24093
- US-A- 4 841 954
- US-A- 5 551 416
- US-A- 6 158 439
- US-A1- 2005 011 514
- US-B2- 6 851 626

## Description

### FIELD OF THE INVENTION

The present invention generally relates to a vibrated medical device for the treatment and relieving of symptoms caused by nasal congestion and sinusitis.

### BACKGROUND OF THE INVENTION

Sinusitis is a medical condition that affects the lives of many millions of people around the world. As much as 15 million people each year develop symptoms of sinusitis, which are often annoying and disturbing a patient to conduct his everyday life. The symptoms of sinusitis usually comprise pain, tenderness, and swelling depending on the affected sinus. Known treatment for sinusitis comprises use of nasal sprays and medication that cause blood vessels to narrow and are aimed to improve sinuses drainage. Other types of sinusitis require the use of antibiotics. However, the known suggested treatments are often not useful. Furthermore, for said medications used have often short-term expediency and most of the medication has undesirable side effects.

There is therefore a need in the art for a simple, safe, relatively cheep, as well as devoid of side effect treatment form that is not surgical. There is further need for a treatment modality that has a high efficacy and a long-term effect on the person inflicted with nasal congestion and/or sinusitis symptoms.

Upper respiratory disorders such as viral upper respiratory tract infections or "common cold", allergic rhinitis, and rhinosinusitis are associated with impairment in mucociliary clearance in the nasal passages. Although the causes of these disorders are varied, they share a common set of nasal symptoms such as rhinorrhea, nasal congestion/blockage, and post-nasal drip. In these conditions the mucous membranes of the nose and paranasal sinuses become irritated, leading to symptoms. In some patients, this irritation is sufficient to hinder the normal drainage of the sinuses into the nasal cavity, resulting in blockage that may lead to additional impaired ciliary activity, intense pressure/pain, and increased likelihood of infection.

Allergic rhinitis (AR) is a condition that results from exposure to allergens, either at specific times of the year (seasonal allergic rhinitis) or year-round (perennial allergic rhinitis). Up to one-half of AR patients suffer from both seasonal as well as perennial AR, approximately one-third suffer from seasonal AR alone and another one-third from perennial AR alone. In either seasonal or perennial AR, the symptoms and treatment approaches are similar. Symptoms most often include nasal congestion or stuffiness, rhinorrhea and nasal itching. Allergic rhinitis affects nearly 150 million people in the world's seven major pharmaceutical markets, and annual sales of prescription products to treat allergic rhinitis are estimated to total more than $4.5 billion worldwide. The treatments currently available include primarily prescription and over-the-counter antihistamines, decongestants and nasal corticosteroids, delivered by nasal sprays, evaporation devices, and ointments.

Upper Respiratory Tract Infections (URTI) and the common cold affect all ages and are uncomfortable conditions with lost work and school days.

US 6,158,439 A discloses a vibrational instrument which is therapeutically beneficial in treating the person suffering from sinus congestion and pressure, and withdrawal due to drug dependency includes a member capable of transmitting vibrational waves through the teeth, jaw and sinus tissue for disposal between teeth of the person. A method of treatment is also provided. WO 93/24093 A discloses a device for the treatment of the cavities in the respiratory passages generates acoustic vibrations, which resonate in the cavities of the respiratory passages and provide a treating action. The most important parts of the device are the signal source, which generates the vibration signals, and an acoustic vibrator, which directs the vibrations into the cavities of the respiratory passages. The treating action of the device is based on the fact that resonating acoustic vibrations loosen mucus and other secretions, massage the mucus membranes and stimulate their circulation, facilitating the draining of mucus and other secretion through natural ducts into the throat and relaxing the muscles connected to the cavities. US 4,841,954 on which the preamble of claim 1 is based describes an oculo-facial massager for simultaneously providing a massaging action to the eye, nose and temple areas. A contoured frame contacts the face of the user and a vibration generating device provides vibration to the frame which is transmitted to the facial muscles contacted by the frame.

### SUMMARY OF THE PRESENT INVENTION

It is the object of the present invention to provide a cost effective device for treating nasal congestion and/or relieving sinusitis symptoms in a patient, comprising attaching means and a vibration generating means. The attaching means are in communication with the patient's head in a location adjacent to said sinuses to be treated. The generating means are adapted to vibrate the attaching means and thus to vibrate the location adjacent to the sinuses.

It is also in the scope of the present invention wherein the device also comprising medicament dispersing means. The device further comprises means for forcing a stream of fluid towards the patient's respiration tracks.

Preferably the fluid is a mixture of air and at least one liquid or gas or powder other than air, and the respiration tracks are selected from the nasal tracks and the pulmonary systems.

### BRIEF DESCRIPTION OF THE FIGURES

In order to understand the invention and to see how it may be implemented in practice, a preferred embodiment will now be described, by way of non-limiting example only, with reference to the accompanying drawing, in which
figure 1 is presenting a schematic illustration of a device (1) with several possible modules according to one embodiment of the present invention;
figure 2 is presenting a lateral view of a device (without the housing's cover) showing some of the device's modules according to another embodiment of the present invention;
figure 3, is presenting a side view of an incorporated vibrating and venting mechanisms actuated by a single electrical motor according to another embodiment of the present invention;
figure 4 is presenting a side view of the venting and inhaling means according to another embodiment of the present invention;
figure 5 is presenting a side view of the housing of the medicament's container according to another embodiment of the present invention;
figure 6 schematically presenting a side view of a disc pulsed inhaler (incomplete mechanism) according to yet another embodiment of the present invention; said device comprising multiple motors, e.g., a vibrator (71) potentially enveloped by a sponge (73), and a disc motor (72) in communication with a fan-like disc (74);
figure 7 schematically presenting a side view of a disc pulsed inhaler (complete mechanism) according to said multiple-motors containing embodiment of the present invention;
figure 8 schematically presenting a side view of the envelope of a disc pulsed inhaler according to said multiple-motors containing embodiment of the present invention; and,
figure 9 schematically presenting a top and front view of the same, only partially covered by its envelope.

### DETAILED DESCRIPTION OF PREFERRED EMBODIMENTS

The following description is provided, alongside all chapters of the present invention, so as to enable any person skilled in the art to make use of said invention and sets forth the best modes contemplated by the inventor of carrying out this invention. Various modifications, however, will remain apparent to those skilled in the art, since the generic principles of the present invention have been defined specifically to provide means and method of treating nasal congestion and/or relieving sinusitis symptoms in a patient, comprising attaching a vibration generating means to the patient head, at a location adjacent to sinuses to be treated, generating a vibration by said vibration generating means, and delivering the same to said patient.

The term **'body portions adjacent to nasal cavity, nasal passageways sinus or sinuses'** refers hereinafter to any location at the head of the patient, and especially to locations selected from the nasal bone, root-, dorsum- or bridge- of the nose, nostrils, frontal bone, temporal bone, maxilla bone, superciliary arch or any combination thereof. The term is further related to the pulmonary system.

In a preferred embodiment of the present invention a stream of fluid is forced towards the patient's respiration tracks. The term forcing a stream of fluid' refers hereinafter to the generation of flowing fluid and its directions either as a contentious flow of fluid or a pulses of the same by means of vents, fans, jets, injectors, compressors, pumping means or other means known in the art adapted to force a fluid towards at least one predetermined location. Preferably, the aforesaid fluid is air. Alternatively, the aforesaid fluid is a mixture of air and at least one liquid or gas other than air.

Inhaling of a dispersed medicament while vibrating the respiratory tracks is thus the core of the present invention.

It is according to another embodiment of the present invention wherein the medicament is introduced to the dispenser by means of a disposable capsule or the like. Said capsule comprises an envelope adapted to be punctured such that at least a limited measure of the medicament contained in said enveloped is forced to flow toward the pulsed column of fluid in a predetermined flux.

According to a further embodiment of the present invention the medicament is contained in an envelope (capsule). The insertion of this capsule may initiate the medicament delivery by means of a micro-switch triggered by the detergent dispensing cabinet when said cabinet is closed. Said capsule may provide coded information for activating the dispensing of the medicament. In another embodiment of the present invention the medicament delivery is provided by a 2D or 3D specific and predetermined fitting mechanism, e.g., fitting by means of shape and sizes. In a further embodiment of the present invention the shape of the capsules inside the inhaler envelope provides key-in-lock information for activating the inhaler.

It is according to another embodiment of the present invention wherein said method additionally comprising heating of said fluid before or in the process of forcing it towards the respiratory tracks. Such a heating step provides for an effective dispersion or evaporation of the fluid, the medicaments or both. It is further acknowledged in this respect that said method may also comprising a step or steps of physical or chemical activating a medicament by reacting it with one or more reactants before or in the process of forcing the same towards the respiratory tracks.

A device for treating nasal congestion and/or relieving sinusitis symptoms in a patient is further presented. This novel device is comprised of both an attaching means and a vibration generating means being either incorporated or interconnected.

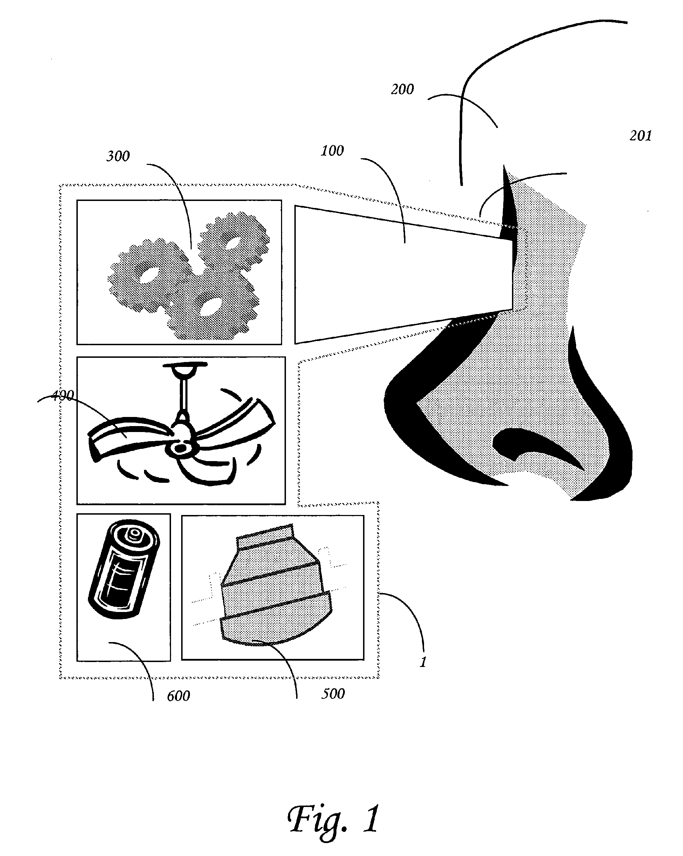

Fig. 1 shows the device (1) according to one embodiment of the present invention comprising ingredients selected from attaching means (100) adapted to attach device (1) to the patient's head (200), especially to the nasal bridge (201); vibrating means (300); means for forcing a stream of fluid (400); medicaments, reactants and/or fluids other than air container (500); power supply (600), and a light housing (See dashed line, 1) or any combination thereof.

The attachment of attaching means (100) to the patients face is preferably provided by physical means, such as clippers, fasteners, vacuum means or a combination thereof. The size and shape of said attaching means is preferably adjustable and expendable. It may be characterized by various shapes, such as clipper-like, glasses or spectacles-like, mask-like or other shapes are utilized. While device (1) may be immobilized to the patent's face without a continuous help of the patient, other modes, requiring the patient to carry the device for a prolong time, are also possible. The proximal end of attaching means (100) may be wide and comprise a relatively wide contacting surface, yet it may alternatively be characterized by a figure-like end, massaging the patients with narrow effecting tips.

A secondary vibrating means are provided according to yet another embodiment of the present invention, wherein said attaching means comprising at least one joint, passively translating a vibration along one axis to movement along at least one other axis.

It is further in the scope of the present invention wherein said proximal portion is heated to a predetermined measure above the ambient temperature, for example up to about 45°C.

Vibration generating means (300) are adapted to generate vibrations, e.g., oscillations, sharp movements etc in a predetermine frequency and intensity protocol. Said protocol is selected in a non-limiting manner from continuous or pulsed, equal or varied vibration characteristics. Said vibrating means, as other ingredients of device, is regulated by the user, (on/Off switch, regulator or program selection know, not shown), and/or by a remote controller. Preferably, the amplitude will range from about 5 vibrations per second (Hertz) to a range greater than about 1,000 Hertz, while each session in the procedure is provided for about 1 to about 15 minutes.

It is according to another embodiment of the present invention wherein the vibrating means comprising an electrical or electro-magnetic motor.

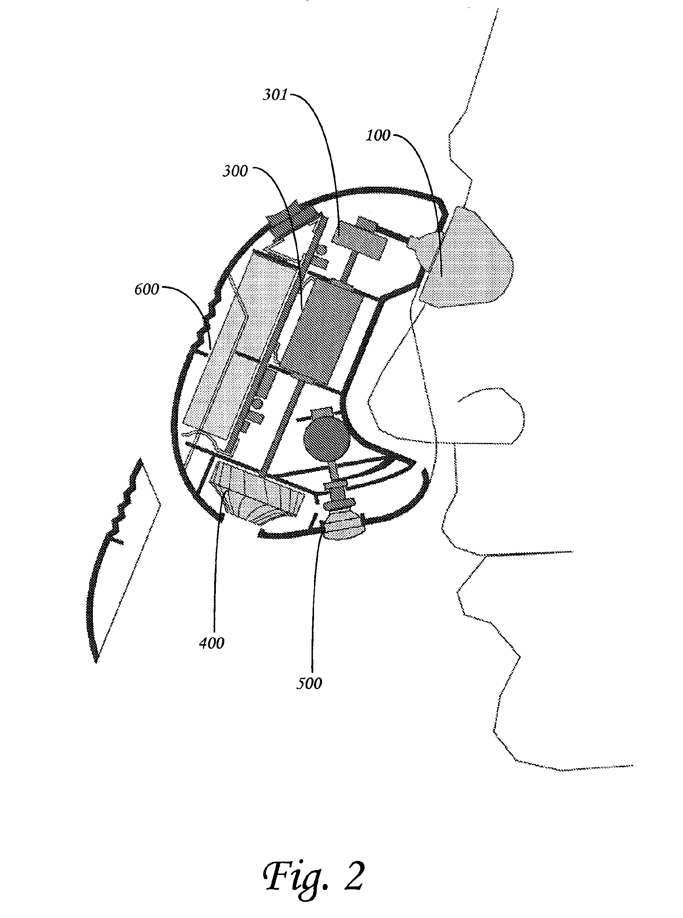

Reference is made now to figure 2, illustrating the various modules of device 1 defined above, wherein vibrating means (300) are interconnected with the attaching means (100) by at least one mutual axle, here, and in a non-limiting manner, a coaxial transmitter (301).

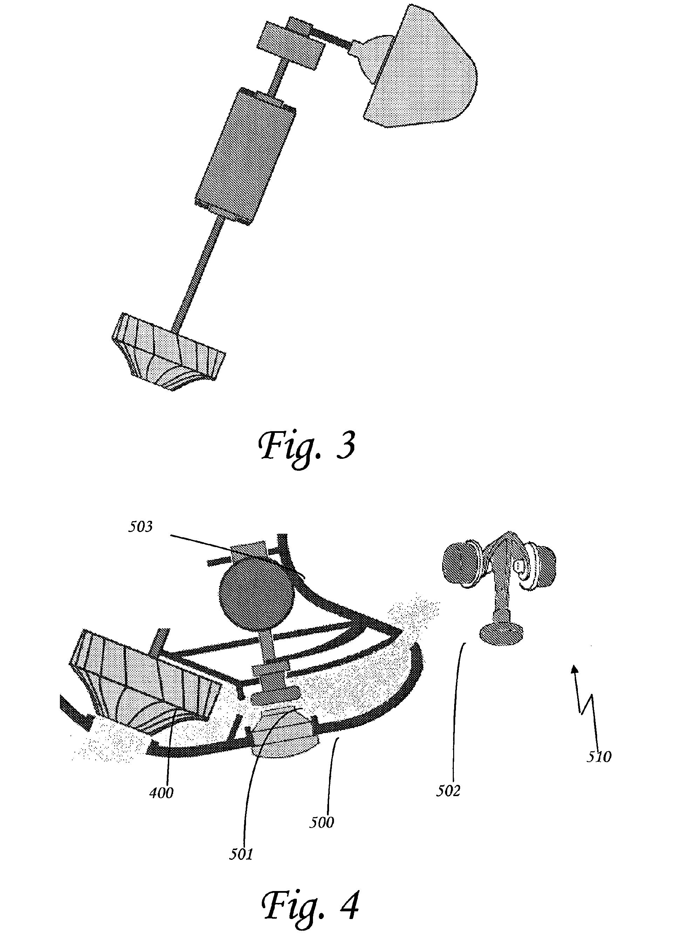

In a preferred embodiment of the present invention the device 1 additionally comprises means for forcing a stream of fluid towards the patient's respiration tracks. Said venting to dispersing means is either separated from the vibrating and attaching means or incorporated tone mechanism. Reference is made thus to figure 3 schematically presenting one electrical engine incorporated in one end to the vibrating means (upper portion) and to a rotating cyclone (lower portion).

According to another embodiment of the present invention the fluid forced towards is air, either at ambient temperature or of lower/higher temperature. The heating is easily provided by forcing the fluid throughout a heated labyrinth, wherein its cooling is provided by wetting it or forcing it to a Peltier device. Alternatively, the fluid forced by the venting mechanism is a mixture of air and at least one liquid, solid powder or gas other than air or powder with air. This heating or cooling means adapted to heat or chill said fluid is preferably provided before or in the process of forcing it towards the respiratory tracks.

It is according to another embodiment of the present invention wherein device 1 additionally comprises means to store medicaments and other fluids and to provide its inhalation by the respiratory tracks of the patients, e.g., via his nasal or oral systems. The term 'medicaments' is hereinafter related in a non-limiting manner to any medicament, fluid, essential oil, volatile compound, etheric oil, terpene, terpanol and either water miscible or water-immiscible extracts, especially oils or extracts of vegetables or fruits or powder. Additionally, said device may also comprising activating means adapted to activate said medicament by reacting it with one or more reactants before or in the process of forcing the same towards the respiratory tracks.

Reference is made now to figure 4, presenting a close look to said inhaling system, wherein rotating cyclone (400) is forcing ambient air towards a thin path (501) such that medicament accommodated in container (500) is dispersed. The fluid, now comprising a predetermined measure of the released medicament (502) is forced towards the client respiratory tracks. Possibly, operating knob (503) provides said release, e.g., by opening said container's cover. The release mechanism (510), here comprising two knobs are also described.

It is further in the scope of the present invention wherein the released medicament (502) is forced towards the client respiratory tracks directly as defined above or indirectly, e.g. via one or more dispensing means being either active or passive. Active dispensing means are selected in a non-limiting manner from medicament dispensers, such as inhalers of Ventolin TM (salbutamol TM) or the like, humidifiers etc. It is thus according to one embodiment of the present invention wherein the dispersed medicaments forced outside the invented device is fed into said active dispensers. Passive dispensing means are either flexible or rigid pipes, tubes and other conducting means adapted to force or purge at least a portion of the dispersed material towards a predetermined, e.g., focused target being either adjacent to the invented device or located in a remote location.

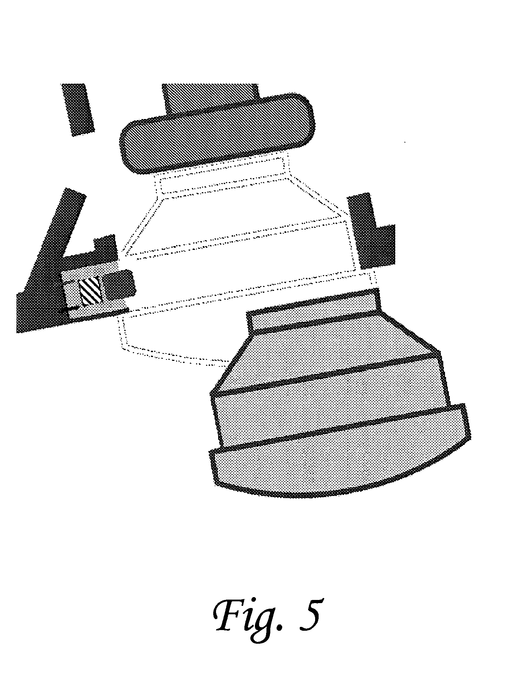

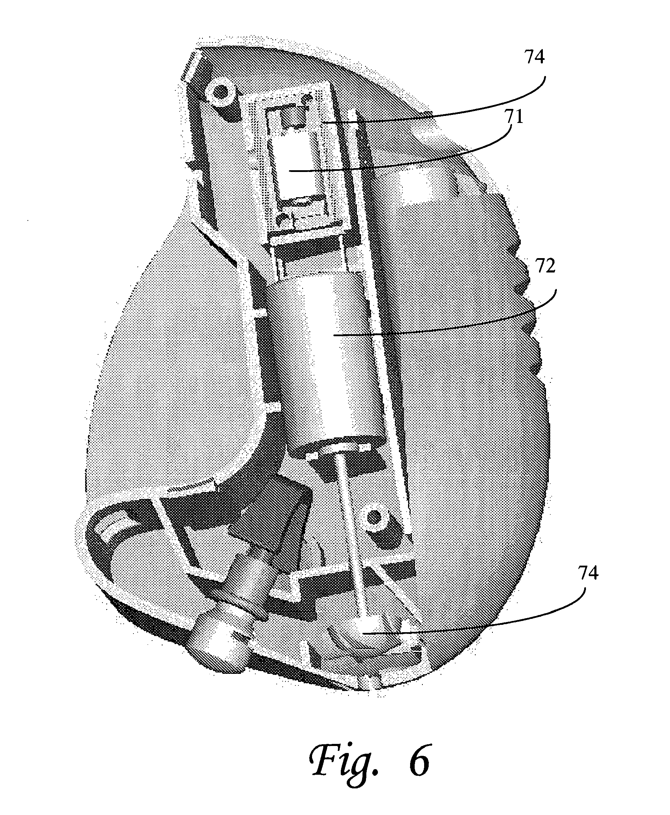

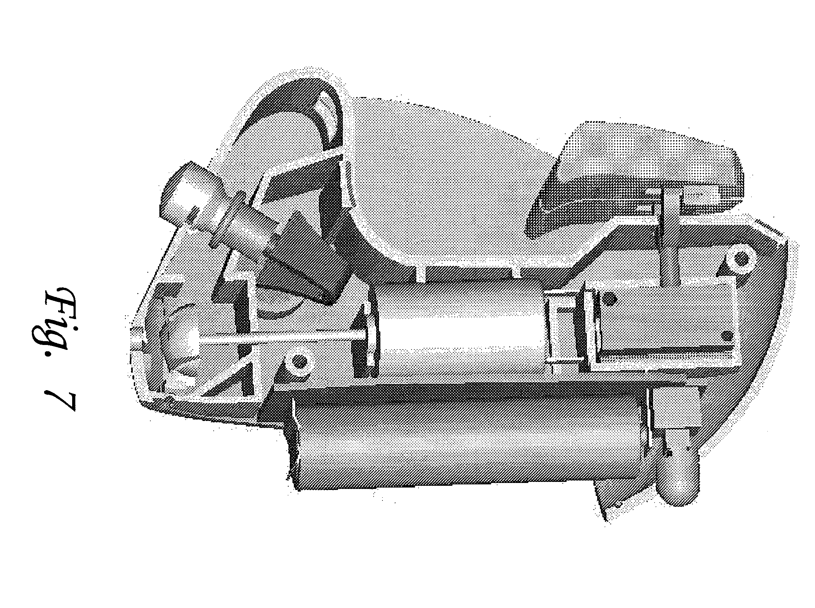

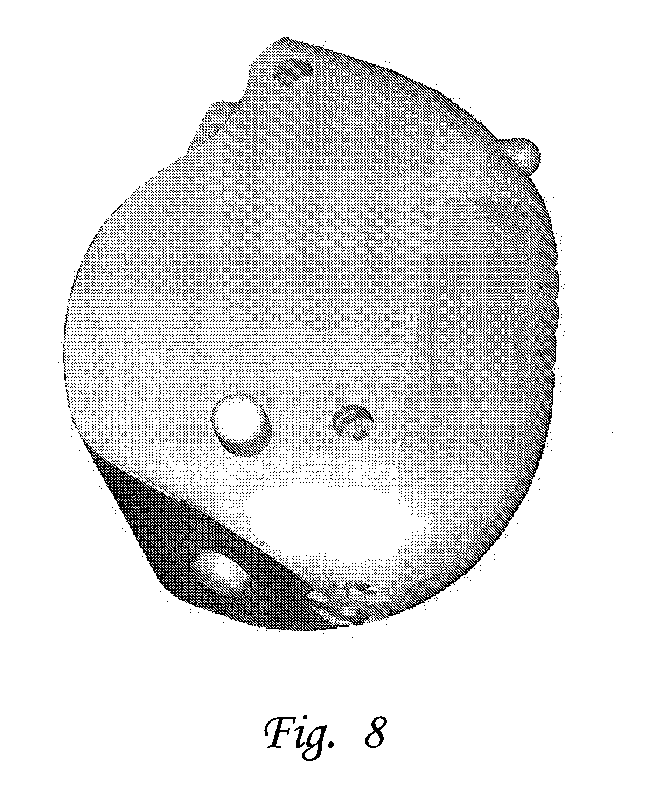

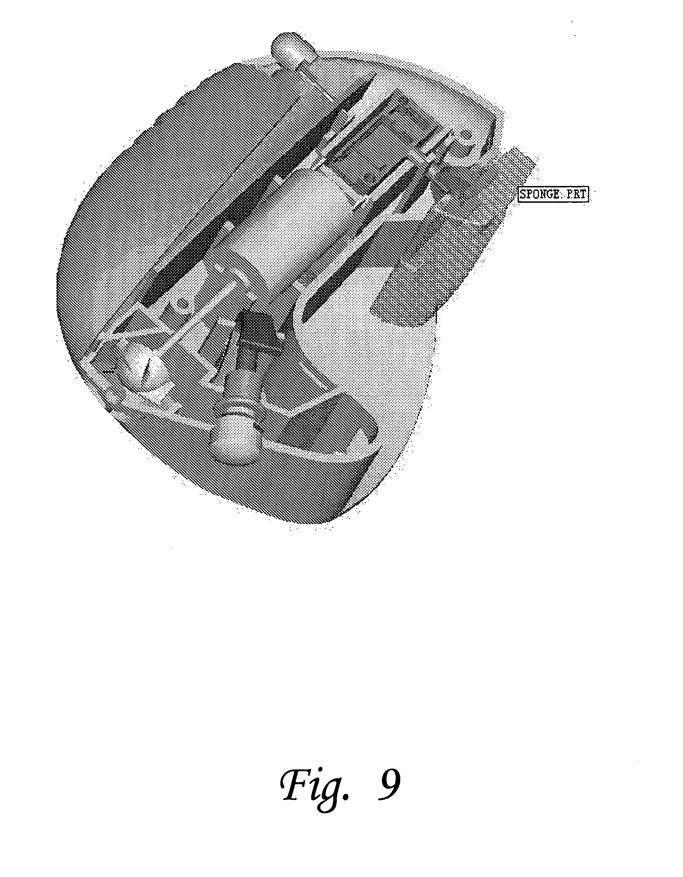

Reference is made now to figure 5, schematically presenting a close look the medicament container (500) in its housing. Said housing and the container's releasing mechanism provides for a quick and easy displacement of the medicament's container. A possible heating device allowing an increase of the dispersion of the medicaments is not shown. Reference is also made now to figure 6-9, illustrating a multiple-motors containing inhaler; wherein figure 6 schematically presents a side view of a disc pulsed inhaler (incomplete mechanism) according to yet another embodiment of the present invention. The portable and cost effective device comprising multiple motors, e.g., a vibrator (71) potentially enveloped by a sponge (73), and a disc motor (72) in communication with a fan-like disc (74). Figure 7 schematically presents a side view of the same (complete mechanism); figure 8 schematically illustrates side view of the envelope of a disc pulsed inhaler and figure 9 schematically discloses a top and front view of the same, only partially covered by its envelope.

## Claims

1. Device (1) for treating nasal congestion and/or relieving sinusitis symptoms in a patient, comprising attaching means (100) and vibration generating means (300); said attaching means (100) adapted to attach said device (1) to the patient's head (200) and are adapted to be in communication with the patient's head (200) in a location adjacent to a nasal cavity, a nasal passageway or sinuses to be treated; said vibration generating means (300) adapted to vibrate said attaching means (100) in order to vibrate the location adjacent to said nasal cavity or sinuses and thus improved mucociliary clearance of secretions in nasal cavity or sinuses
**characterized in that**
said device (1) further comprises means (400) of forcing a stream of fluid towards the patient's respiration tracks.

2. The device (1) according to claim 1, additionally comprising medicament dispersing means.

3. The device (1) according to claim 2, wherein the medicament is at least partially contained in a capsule.

4. The device (1) according to claim 2, wherein the medicament delivery is provided by a two-dimensional or three-dimensional specific and predetermined fitting mechanism.

5. The device (1) according to claim 1, wherein the vibration generating means (300) comprises a motor (72), a power supply (600) and a rotor in communication with a coaxially rotating axle (301), said rotation being translated into a vibration and further being delivered to a body portion adjacent to the sinuses of the patient.

6. The device (1) according to claim 1, wherein the vibration generating means (300) comprises an electrical or electro-magnetic motor.

7. The device (1) according to claim 1, wherein the fluid is air or a mixture of air and at least one liquid, gas or solid powder other than air.

8. The device (1) according to claim 1, additionally comprising heating means adapted to heat said fluid before or in the process of forcing it towards the respiratory tracts.

9. The device (1) according to claim 1, additionally comprising activating means adapted to activate a medicament by reacting it with one or more reactants before or in the process of forcing the same towards the respiratory tracts.

10. The device (1) according to claim 2, wherein the dispersing means being either active or passive means, adapted to force or purge at least a portion of the dispersed material towards a predetermined, e.g., focused target being either adjacent to the invented device (1) or located in a remote location.

## Patentansprüche

1. Vorrichtung (1) zur Behandlung einer nasalen Kongestion und/oder zur Behebung von Sinusitissymptomen bei einem Patienten, umfassend Mittel zum Anfügen (100) sowie Mittel zur Erzeugung von Vibrationen (300); wobei die Mittel zum Anfügen (100) dazu ausgebildet sind, die Vorrichtung (1) an den Kopf (200) des Patienten anzufügen und mit dem Kopf (200) des Patienten in Verbindung zu stehen, an einem Ort neben einer nasalen Kavität, einer nasalen Passage oder neben zu behandelnden Sinussen; und wobei die Mittel zur Erzeugung von Vibrationen (300) dazu ausgebildet sind, die Mittel zum Anfügen (100) in Vibration zu versetzen, sodass der Ort neben der nasalen Kavität oder den Sinussen vibriert und somit die mukoziliäre Freiheit von Sekreten in der nasalen Kavität oder den Sinussen zu verbessern,
**dadurch gekennzeichnet, dass**
die Vorrichtung (1) weiterhin Mittel (400) zum Erzwingen eines Fluidstroms zu den Atemwegen des Patienten umfasst.

2. Die Vorrichtung (1) gesäß Anspruch 1, weiterhin umfassend ein Medikamentenausgabesystem.

3. Die Vorrichtung (1) gemäß Anspruch 1, wobei das Medikament zumindest teilweise in einer Kapsel ist.

4. Die Vorrichtung (1) gemäß Anspruch 2, wobei die Medikamentenausgabe einen zweidimensionalen oder einen dreidimensionalen, spezifischen und vordefinierten Passmechanismus umfasst.

5. Die Vorrichtung (1) gemäß Anspruch 1, wobei die Mittel zur Erzeugung von Vibrationen (300) einen Motor (72), eine Energieversorgung (600) und einen Rotor, in Verbindung mit einer koaxialen rotierenden Achse (301), umfassen, wobei die Rotation in eine Vibration übersetzt ist und desweiteren an einem Körperbereich abgegeben wird, welcher neben den Sinussen eines Patienten liegt.

6. Die Vorrichtung (1) gemäß Anspruch 1, wobei die Mittel zur Erzeugung von Vibrationen (300) einen elektrischen oder elektromagnetischen Motor umfassen.

7. Die Vorrichtung (1) gemäß Anspruch 1, wobei das Fluid Luft ist oder eine Mischung von Luft und mindestens einer von Luft verschiedenen Flüssigkeit, einem von Luft verschiedenem Gas oder einem festen Pulver.

8. Die Vorrichtung (1) gemäß Anspruch 1, weiterhin umfassend Mittel zum Heizen, ausgebildet zum Heizen des Fluids vor oder während des Prozesses des Erzwingens des Fluidstroms zu den Atemwegen.

9. Die Vorrichtung (1) gemäß Anspruch 1, weiterhin umfassend Mittel zur Aktivierung, ausgebildet zur Aktivierung eines Medikaments durch Reaktion mit einer oder mehreren Reaktanzien vor oder während des Prozesses dieses zu den Atemwegen zu zwingen.

10. Die Vorrichtung (1) gemäß Anspruch 2, wobei die Mittel zur Ausgabe entweder aktiv oder passiv sind und zum Zwingen oder Spülen mindestens eines Teils des ausgegebenen Materials zu einem vorbestimmten, zum Beispiel fokussierten Ziel ausgebildet sind, welches sich entweder neben der erfindungsgemäßen Vorrichtung (1) oder sich an einem entfernten Ort befindet.

## Revendications

1. Dispositif (1) pour traiter les congestions nasales et/ou pour soulager les symptômes de la sinusite dans un patient, comprenant un moyen de fixation (100) et un moyen de génération de vibrations (300), ledit moyen de fixation (100) étant conçu pour fixer ledit dispositif (1) à la tête du patient (200) et étant conçu pour une mise en communication avec la tête du patient (200) à un endroit situé en position adjacente à une cavité nasale, une voie nasale ou des sinus à traiter, ledit moyen générant des vibrations (300) étant conçu pour faire vibrer ledit moyen de fixation (100) afin de faire vibrer l'endroit situé en position adjacente à ladite cavité nasale ou auxdits sinus et ainsi améliorer la clairance mucocillaire des sécrétions dans la cavité nasale ou dans les sinus, **caractérisé en ce que** ledit dispositif (1) comprend en outre un moyen (400) pour forcer un courant de fluide en direction des voies respiratoires du patient.

2. Dispositif (1) selon la revendication 1, comprenant en outre un moyen de distribution de médicament.

3. Dispositif (1) selon la revendication 2, dans lequel le médicament est contenu au moins en partie dans une capsule.

4. Dispositif (1) selon la revendication 2, dans lequel la distribution du médicament est fournie par un mécanisme d'emboîtement spécifique et prédéterminé en deux dimensions ou en trois dimensions.

5. Dispositif (1) selon la revendication 1, dans lequel le moyen générant des vibrations (300) comprend un moteur (72), une alimentation électrique (600) et un rotor mis en communication avec un essieu à rotation coaxiale (301), ladite rotation se transformant en une vibration et étant en outre distribuée à une portion du corps en position adjacente aux sinus du patient.

6. Dispositif (1) selon la revendication 1, dans lequel le moyen générant des vibrations (300) comprend un moteur électrique ou électromagnétique.

7. Dispositif (1) selon la revendication 1, dans lequel le fluide est de l'air ou un mélange d'air et d'au moins un élément choisi parmi un liquide, un gaz ou une poudre solide, différent de l'air.

8. Dispositif (1) selon la revendication 1, comprenant en outre un moyen de chauffage conçu pour chauffer ledit fluide avant qu'il ne soit forcé ou pendant qu'il est forcé en direction des voies respiratoires.

9. Dispositif (1) selon la revendication 1, comprenant en outre un moyen d'activation conçu pour activer un médicament en le faisant réagir avec un ou plusieurs réactifs avant qu'il ne soit forcé ou pendant qu'il est forcé en direction des voies respiratoires.

10. Dispositif (1) selon la revendication 2, dans lequel le moyen de distribution est un moyen actif ou passif conçu pour forcer ou pour purger au moins une portion de la matière distribuée en direction d'une cible prédéterminée, par exemple focalisée, soit en position adjacente au dispositif (1) selon l'invention, soit à un endroit situé à distance.
